# EUROPEAN PATENT SPECIFICATION

(11) **EP 4 395 845 B1**
(45) Date of publication and mention of the grant of the patent: **02.10.2024**
(21) Application number: 22786853.6
(22) Date of filing: 02.09.2022
(51) Int. Cl.: A61L 27/34, A61L 27/50, A61L 27/54

(54) **A METHOD FOR MANUFACTURING DENTAL IMPLANTS HAVING HYDROPHILIC SURFACE**
VERFAHREN ZUR HERSTELLUNG VON ZAHNIMPLANTATEN MIT HYDROPHILER OBERFLÄCHE
PROCÉDÉ DE FABRICATION D'IMPLANTS DENTAIRES AYANT UNE SURFACE HYDROPHILE

(30) Priority: 02.09.2021 TR 202113826
(43) Date of publication of application: 10.07.2024
(73) Proprietor: Yildiz Teknik Universitesi, 34220 Istanbul (TR)
(72) Inventor: HAZAR, Afife Binnaz, 34220 Istanbul (TR); SAZCI, Oguler, Istanbul (TR); GENC, Gulcin, Istanbul (TR)
(74) Representative: Genç Ilhan, Oznur
(86) International application number: PCT/TR2022/050941
(87) International publication number: WO 2023/033775

(56) References cited:
- LI SHENGXI ET AL: "Properties of Electropolymerized Dopamine and Its Analogues", LANGMUIR, vol. 35, no. 5, 23 August 2018 (2018-08-23), US, pages 1119 - 1125, XP093006113, ISSN: 0743-7463, Retrieved from the Internet <URL:https://pubs.acs.org/doi/pdf/10.1021/acs.langmuir.8b01444> DOI: 10.1021/acs.langmuir.8b01444
- LI YAOXU ET AL: "Application of polydopamine on the implant surface modification", POLYMER BULLETIN, SPRINGER, HEIDELBERG, DE, vol. 79, no. 8, 21 June 2021 (2021-06-21), pages 5613 - 5633, XP037895282, ISSN: 0170-0839, [retrieved on 20210621], DOI: 10.1007/S00289-021-03793-9
- LEE JUNG-JUN ET AL: "Effects of polydopamine coating on the bioactivity of titanium for dental implants", INTERNATIONAL JOURNAL OF PRECISION ENGINEERING AND MANUFACTURING, vol. 15, no. 8, 1 August 2014 (2014-08-01), Springer, pages 1647 - 1655, XP055858072, ISSN: 2234-7593, Retrieved from the Internet <URL:http://link.springer.com/article/10.1007/s12541-014-0515-6/fulltext.html> DOI: 10.1007/s12541-014-0515-6
- WANG JIN-LEI ET AL: "Electropolymerization of dopamine for surface modification of complex-shaped cardiovascular stents", BIOMATERIALS, ELSEVIER, AMSTERDAM, NL, vol. 35, no. 27, 12 June 2014 (2014-06-12), pages 7679 - 7689, XP028859286, ISSN: 0142-9612, DOI: 10.1016/J.BIOMATERIALS.2014.05.047

## Description

### Technical Field of the Invention

The present invention relates to dental implants. In particular, the present invention relates to dental implants with low surface energy.

### Background Art

A dental implant is an artificial tooth root that is inserted in the jawbone to restore the function of the lost tooth and impaired aesthetics. Although dental implant treatment is highly successful today, there are still serious losses occurring due to some complications such as perimucositis and peri-implantitis.

Osseointegration is defined as the structural and functional connection between living bone and the load-bearing intraosseous implant. An insufficient osseointegration of the implant with the bone, impairment of the bacterial balance after unsuccessful osseointegration, infection due to bone loss and soft tissues around the implant are the most important causes of dental implant losses. The occurrence of such complications is largely dependent upon the material type and surface properties of the implant, as well as the patient's awareness of oral hygiene and the experience of the doctor.

Microstructural changes on the surface of the dental implant are effective in the response of tissues and cells to the implant. In order to establish a good osseointegration between the implant and the bone tissue, physical, chemical, physicochemical treatments and/or combinations thereof are applied to the surface. With a good osseointegration, the possibility of inflammation in the tissues around the implant is reduced. However, existing surface treatments are not sufficient to prevent such diseases as perimucositis and peri-implantitis. In general, it is apparent from information obtained from case studies and clinical studies that all the methods above may cause problems in implant-tissue compatibility.

The main factor underlying this invention is the fact that intraoral inflammations caused by implants are still experienced today despite the existing surface studies. Peri-implant diseases that occur due to three main factors, namely the patient, the doctor and the material of the implant, if it is accepted that the patient performs a regular oral care and the doctor performs the correct treatment, result from the implant material.

Li Shengxi et al. "Properties of Electropolymerized Dopamine and Its Analogues" Langmuir, vol. 35, no. 5, pages 1119-1125, discloses a study of electropolymerization kinetics, film thickness, stability, and antifouling properties of polydopamine (PDA). As compared to self-polymerization, the electropolymerization of dopamine results in a higher deposition rate, which can be achieved in acidic solutions, suitable for alkaline-sensitive materials, and also avoids aggregation by increasing the surface roughness. The PDA films obtained from the electropolymerization method have been used for surface modification of a variety of biomaterials.

### Objects of the Invention

Primary object of the present invention is to eliminate the deficiencies of the prior art. Another object of the present invention is to increase the hydrophilicity of the dental implant surfaces by a fast and cost-effective method that is easy to monitor and control.

### Summary of the Invention

Given that there is currently not available a wide range of options in the selection of the base material for the implant, it is sought to solve the abovementioned problem of the prior art through the improvement of the implant surface.

In the context of the present invention, it is to increase the surface energy and improve the bone-implant compatibility due to an increase in the surface wettability.

The method comprises the following sequential steps:
a) subjecting the surface to a pre-treatment for removing the oxide layer; wherein the implant is made of Ti-6Al-4V-ELI and wherein the surface of the implant is subjected to a constant potential of -3 V for 30 seconds;
b) coating said pre-treated implant surface with a polymer layer by means of electropolymerization, in particular by means of a potential applied between a working electrode in an electrolytic cell and a counter electrode, wherein the substrate to be coated is coupled to the electrolytic cell as the working electrode.

For biocompatibility, said polymer may preferably be, for example, a polydopamine (PDA) biopolymer.

The pre-treatment in step a of the method may comprise subjecting the surface to a treatment step to provide it with a roughness value of 0.2 micrometer or higher. Thus, the surface area is extended. Said roughness value can also be achieved by a "roughening" process.

The roughening process may, for example, be carried out by sandblasting. The roughness value may, for example, be in the range of 0.2 to 0.5 micrometers, which can be considered to mean the application of "machine surface finish".

With the removal of the oxide layer in the method, the surface is rendered more conductive and the efficiency of the coating process is increased. Pre-treatment also increases bone development by imparting roughness to the surface. With the deposition of PDA on the pre-treated surface (i.e., coating the surface with PDA), the surface energy is increased, thus the wettability of the surface is improved and bone-implant compatibility is enhanced.

With the coating times of up to 2 hours, a hydrophilicity below 50° was achieved.

The present invention provides a method for increasing the hydrophilicity of a surface of an intracorporeal implant made of conductive material. The method comprises a step of coating said implant with a polymer layer by means of electropolymerization.

In a preferred embodiment of the method, step (b) of coating the implant with the polymer layer comprises the following:
i. immersing the implant as a working electrode in an electrolyte developed to provide a source of monomer;
ii. coating the implant by means of electropolymerization, in the presence of a reference electrode immersed in the electrolyte and a counter electrode, by applying voltage to said working electrode.

A preferred embodiment of the method comprises applying cyclic voltammetry in electropolymerization in accordance with the following parameters:
- a voltage in the range of -1.5 V to +1.5 V;
- a scan rate in the range of 0.02 V/s to 0.5 V/s (preferably 0.1 V/s, as implemented in exemplary experiments);
- a number of cycles in the range of 5 to 100 (preferably 50 cycles, as implemented in the exemplary experiments).

The method preferably comprises selecting an electropolymerization time in the range of 30 minutes to 2 hours.

The method preferably comprises using a dopamine-containing monomer as the monomer for coating with said polymer layer. Thus, a PDA layer is achieved on the implant surface. The dopamine-containing monomer may be dopamine HCl.

A preferred embodiment of the method may comprise adjusting the monomer concentration in the electrolyte to be 1 mg/mL at the start of the electropolymerization, and preparing the electrolyte to be a buffered conductive solution. The electrolyte may be buffered to have a pH of 7.4.

In an exemplary embodiment of the method, Ag-AgCl can be used as the electrode material and Pt can be used as the counter electrode material.

### Brief Description of the Drawings

The present invention is exemplified in the accompanying drawings for better understanding thereof, whose brief explanations given below, which examples are only illustrative of the embodiments of the present invention and are not intended to limit other embodiments and general functions that provide the solution of the technical problem.
Figure 1 is a view of a contact angle, as interpreted in Example 4, for a substrate sample (Ti-6Al-4V-ELI disc sample) with no coating applied through electropolymerization.
Figure 2 is a view of a contact angle of the PDA coated surface (surface of the PDA coated Ti-6Al-4V-ELI disc sample) by applying electropolymerization in the context of example 2, as interpreted in Example 4, in order to observe the effect of the inventive improvement.

### Detailed Description of the Invention

The present invention is explained in detail below with reference to the drawings, whose brief explanations are given above.

Within the scope of the present invention, it is possible to obtain polymer-coated implants in a controlled and rapid manner by applying a polydopamine coating on conductive surfaces through electropolymerization method. Accordingly, a dental implant having a polydopamine coated surface is obtained through electropolymerization method.

The electropolymerization method can be applied to any conductive surface.

The electropolymerization method, by means of a potential applied between a working electrode in an electrolytic cell and a counter electrode, allows oxidation and reduction reactions to take place in the solution, thereby enabling the formation of a coating layer (PDA coating layer) on the surface of a substrate (sample, in the present invention: medical implant, especially dental implant) inserted into the environment. While performing coating through electropolymerization method, the electrolytic cell is preferably coupled with a potentiostat device. The potentiostat device is used to keep the potential, i.e., the voltage value, constant between the working electrode and the reference electrode. The substrate (sample) to be coated is coupled with the electrolytic cell as a "working electrode".

Ag/AgCl can be used as the reference electrode and platinum can be used as the counter electrode. A change in the reference electrode causes a change in the numerical values of the results obtained, but does not result in any change in their interpretation. Therefore, different materials can be selected as reference electrode and counter electrode.

In the context of present invention, it is possible and preferred to apply cyclic voltammetry (CV) in performing the electropolymerization. In cyclic voltammetry, a potential that changes in a negative or positive direction (e.g., increasing in absolute value) is applied on the working electrode, in a predetermined range of values. Thus, current values depending on the changing potential value are obtained. If the current values are monitored throughout the coating process, it allows making interpretation about the progress of the coating process. A decrease observed in the current value indicates that the conductivity of the surface has decreased and non-conductive polydopamine has been successfully coated on the surface. Therefore, it is possible to precisely monitor the performance of the inventive method.

The controllability of the parameters in the electropolymerization method is higher than the traditional immersion coating method. In addition, the time required for performing the coating through electropolymerization method is shorter than that in the prior art methods. Therefore, the method of the invention is attractive both in terms of accuracy and precision, and speed-based economic advantage, and it has a high applicability to the industry.

The following examples are just provided for better understanding of the invention and are not intended to limit the scope of intended protection.

### EXAMPLES

Next, the steps of performing the coating process on an exemplary disc-shaped substrate made of Ti-6Al-4V-ELI are exemplified in order to demonstrate the concept set out by the invention.

### EXAMPLE 1:

An exemplary substrate having a conductive surface is selected in preparation for performing the proper coating process. The surface of the substrate was exposed to a constant potential in order to remove the oxide layer. A constant potential of -3 V was applied for 30 seconds. Thus, a substrate with a surface having high conductivity that is suitable for a highly efficient electropolymerization was obtained.

### EXAMPLE 2:

The substrate used in these exemplary experiments is a sample manufactured from Ti-6Al-4V-ELI, which is suitable for use in medical and especially dental implants, as a material sample having a conductive surface. In order to facilitate the measurement of contact angle after the coating process, the sample was selected to have a flat surface, thus to be in the form of a disk.

The substrate that was pre-treated to remove the oxide layer in Example 1 was coated by means of electropolymerization method. Cyclic voltammetry (in short: CV) was selected as the electropolymerization method. The coating process comprises the following:
i. immersing the substrate as a working electrode in an electrolyte (coating solution) developed to provide a source of dopamine (monomer);
ii. coating the substrate by means of electropolymerization, in the presence of a reference electrode immersed in the electrolyte and a counter electrode, by applying voltage to said working electrode.

With electropolymerization, a source of dopamine (here: dopamine HCl) was selected as the monomer, thereby obtaining a PDA layer as the polymer layer covering the substrate surface.

### EXAMPLE 3: Elements and parameters used as examples in electropolymerization

In the electropolymerization process in Example 2, the following were preferred as the appropriate element and parameter values:

Dopamine HCl was selected as the dopamine source. Accordingly, the electrolyte is developed as follows:
- the concentration (initial concentration) of the monomer (here: dopamine HCl) in the electrolyte at the start of the electropolymerization is, for example, 1 mg/mL;
- the electrolyte is a buffered conductive solution having, as an example/suitable value, a pH of 7.4.

Parameter values used and preferred in the voltage application are as follows:
- applying cyclic voltammetry (CV);
- the applied voltage (potential value) range is preferably in the range of -1.5 V to +1.5 V (a range of -1 V to +1 V is applied in this example);
- the voltage change rate (scan rate) is preferably in the range of 0.02 V/s to 0.5 V/s, for example/preferably 0.1 V/s (0.1 V/s is applied in this example);
- the number of cycles is, for example, in the range of 5 to 100 cycles, for example/preferably 50 cycles (50 cycles are applied in this example);
- the electropolymerization time is preferably in the range of 30 minutes to 2 hours.

The electrolyte (coating solution) used in this laboratory-scale exemplary experiment was prepared, for example, in a volume of 40 mL.

Ag-AgCl was used as the reference electrode material and Pt was used as the counter electrode material.

In the exemplary experiment performed at laboratory scale, the electropolymerization coating process was carried out in a three-edged container (balloon) as an electrolytic cell (in terms of having suitable inlets for the reference electrode, counter electrode, and anode).

The electropolymerization was optionally carried out under/in the presence of nitrogen or oxygen (or under/in the presence of air being a mixture of these).

After completion of the electropolymerization process, the substrate (sample) was removed from the electrolyte (coating solution), rinsed and then dried. The rinsing process was optionally carried out in an ultrasonic bath using ultrapure water for 15 minutes. The drying process was optionally carried out in nitrogen environment.

### EXAMPLE 4:

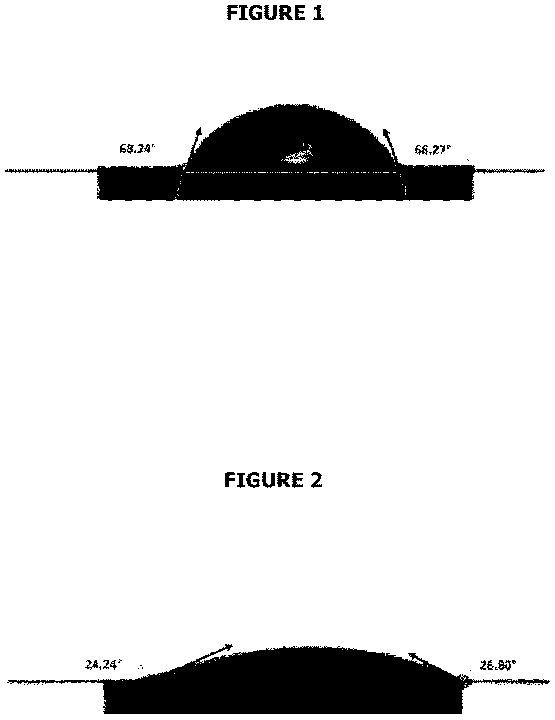

An image of a contact angle is taken for a substrate surface (a sample of Ti-6Al-4V-ELI disc without PDA coating) where the pre-treatment for oxide removal in Example 1 and the electropolymerization coating in Example 2 have not been applied, which is presented in Figure 1. The contact angle on the uncoated surface was measured as 68.26° (an average of 68.24° and 68.27°) and the hydrophilicity level of said surface was considered as a reference.

In order to observe the effect of the improvement of the invention, an image of a contact angle of the PDA-coated substrate which is subjected to electropolymerization (i.e., the PDA-coated Ti-6Al-4V-ELI disk sample) is taken in the context of example 2 (at a potential value from -1 to +1 V, a scan rate of 0.1 V/s, 50 cycles) and presented in Figure 2. With the method of the invention, the contact angle was measured as 25.52° (an average of 24.24° and 26.80°) on the PDA coated surface. Therefore, it was determined that the hydrophilicity level of the surface was increased, when compared to the reference level, which was subjected to pre-treatment for oxide removal under constant tension, and then coated with PDA.

In summary, according to the contact angle measurements presented in Figure 1 and Figure 2, with the PDA coating, the contact angle was reduced from 68.26° to 25.52° and hydrophilicity was increased.

## Claims

1. A method for increasing the hydrophilicity of a surface of an intracorporeal implant made of a conductive material, comprising the following steps:
a) subjecting the surface of the implant to a pre-treatment for removing the oxide layer, wherein the implant is made of Ti-6Al-4V-ELI and wherein the surface of the implant is subjected to a constant potential of -3 V for 30 seconds;
b) coating said pre-treated implant surface with a polymer layer by means of electropolymerization, in particular by means of a potential applied between a working electrode in an electrolytic cell and a counter electrode, wherein the substrate to be coated is coupled to the electrolytic cell as the working electrode

2. The method of claim 1, wherein in the pre-treatment in step a, the surface is subjected to a treatment step to provide it with a roughness value of 0.2 micrometer or higher.

3. The method of claim 2, wherein in the pre-treatment in step a, the surface is subjected to a treatment step to provide it with a roughness value in the range of 0.2 to 0.5 micrometer.

4. The method of any one of claims 1-3, wherein step (b) includes the following:
i. immersing the implant as a working electrode in an electrolyte developed to provide a source of monomer;
ii. coating the implant by means of electropolymerization, in the presence of a reference electrode immersed in the electrolyte and a counter electrode, by applying voltage to said working electrode.

5. The method of claim 4, comprising applying cyclic voltammetry in electropolymerization in accordance with the following parameters: a voltage in the range of -1.5 V to +1.5 V, a scan rate in the range of 0.02 V/s to 0.5 V/s, and a cycle number in the range of 5 to 100.

6. The method of claim 5, comprising selecting an electropolymerization time in the range of 30 minutes to 2 hours.

7. The method of any one of claim 5 or 6, wherein the scan rate is 0.1 V/s.

8. The method of any one of claims 1 to 7, comprising using a dopamine-containing monomer as a monomer for coating with said polymer layer.

9. The method according to claim 9, comprising using dopamine HCl as a monomer.

10. The method of any one of claims 5 to 9, comprising adjusting the monomer concentration in the electrolyte to be 1 mg/mL at the start of the electropolymerization, and preparing the electrolyte to be a buffered conductive solution.

11. The method of claim 10, wherein the electrolyte is buffered to a pH of 7.4.

12. The method of any one of claims 5 to 11, wherein Ag-AgCl is used as the electrode material and Pt is used as the counter electrode material.

## Patentansprüche

1. Verfahren zur Erhöhung der Hydrophilie einer Oberfläche eines intrakorporalen Implantats aus einem leitfähigen Material, umfassend die folgenden Schritte:
a) Unterziehen der Oberfläche des Implantats einer Vorbehandlung zum Entfernen der Oxidschicht , wobei das Implantat aus Ti-6Al-4V-ELI hergestellt ist und wobei die Oberfläche des Implantats einem konstanten Potential von -3 V für 30 Sekunden unterzogen wird;
b) Beschichten der vorbehandelten Implantatoberfläche mit einer Polymerschicht durch Elektropolymerisation, insbesondere durch Anlegen eines Potentials zwischen einer Arbeitselektrode in einer Elektrolysezelle und einer Gegenelektrode, wobei das zu beschichtende Substrat als Arbeitselektrode mit der Elektrolysezelle verbunden ist

2. Verfahren nach Anspruch 1, wobei bei der Vorbehandlung in Schritt a die Oberfläche einem Behandlungsschritt unterzogen wird, um sie mit einem Rauhigkeitswert von 0,2 Mikrometer oder mehr zu versehen.

3. Verfahren nach Anspruch 2, wobei bei der Vorbehandlung in Schritt a die Oberfläche einem Behandlungsschritt unterzogen wird, um sie mit einem Rauhigkeitswert im Bereich von 0,2 bis 0,5 Mikrometer zu versehen.

4. Verfahren nach einem der Ansprüche 1 bis 3, wobei Schritt (b) Folgendes umfasst:
i. Eintauchen des Implantats als Arbeitselektrode in einen Elektrolyten, der entwickelt wurde, um eine Monomerquelle bereitzustellen;
ii. Beschichtung des Implantats durch Elektropolymerisation in Gegenwart einer in den Elektrolyten eingetauchten Bezugselektrode und einer Gegenelektrode durch Anlegen einer Spannung an die Arbeitselektrode.

5. Verfahren nach Anspruch 4, umfassend die Anwendung der zyklischen Voltammetrie bei der Elektropolymerisation in Übereinstimmung mit den folgenden Parametern: eine Spannung im Bereich von -1,5 V bis +1,5 V, eine Abtastrate im Bereich von 0,02 V/s bis 0,5 V/s und eine Zykluszahl im Bereich von 5 bis 100.

6. Verfahren nach Anspruch 5, umfassend die Auswahl einer Elektropolymerisationszeit im Bereich von 30 Minuten bis 2 Stunden.

7. Verfahren nach einem der Ansprüche 5 oder 6, wobei die Abtastrate 0,1 V/s beträgt.

8. Verfahren nach einem der Ansprüche 1 bis 7, umfassend die Verwendung eines dopaminhaltigen Monomers als Monomer zum Beschichten mit der Polymerschicht.

9. Verfahren nach Anspruch 9, bei dem Dopamin-HCl als Monomer verwendet wird.

10. Verfahren nach einem der Ansprüche 5 bis 9, umfassend das Einstellen der Monomerkonzentration im Elektrolyten auf 1 mg/ml zu Beginn der Elektropolymerisation und das Herstellen des Elektrolyten als gepufferte leitfähige Lösung.

11. Verfahren nach Anspruch 10, wobei der Elektrolyt auf einen pH-Wert von 7,4 gepuffert ist.

12. Verfahren nach einem der Ansprüche 5 bis 11, wobei Ag-AgCl als Elektrodenmaterial und Pt als Gegenelektrodenmaterial verwendet wird.

## Revendications

1. - Procédé pour augmenter le caractère hydrophile d'une surface d'un implant intracorporel fait d'un matériau conducteur, comprenant les étapes suivantes :
a) soumettre la surface de l'implant à un prétraitement pour retirer la couche d'oxyde, l'implant étant fait de Ti-6Al-4V-ELI et la surface de l'implant étant soumise à un potentiel constant de -3 V pendant 30 secondes ;
b) revêtir ladite surface d'implant prétraitée par une couche de polymère au moyen d'une électropolymérisation, en particulier au moyen d'un potentiel appliqué entre une électrode de travail dans une cellule électrolytique et une contre-électrode, le substrat à revêtir étant couplé à la cellule électrolytique en tant qu'électrode de travail.

2. - Procédé selon la revendication 1, dans lequel, lors du prétraitement à l'étape a, la surface est soumise à une étape de traitement pour lui conférer une valeur de rugosité de 0,2 micromètre ou plus.

3. - Procédé selon la revendication 2, dans lequel, lors du prétraitement de l'étape a, la surface est soumise à une étape de traitement pour lui conférer une valeur de rugosité dans la plage de 0,2 à 0,5 micromètre.

4. - Procédé selon l'une quelconque des revendications 1 à 3, dans lequel l'étape (b) comprend ce qui suit :
i. immerger l'implant en tant qu'électrode de travail dans un électrolyte développé pour fournir une source de monomère ;
ii. revêtir l'implant au moyen d'une électropolymérisation, en présence d'une électrode de référence immergée dans l'électrolyte et d'une contre-électrode, par application d'une tension à ladite électrode de travail.

5. - Procédé selon la revendication 4, comprenant l'application d'une voltammétrie cyclique dans l'électropolymérisation conformément aux paramètres suivants : une tension dans la plage de -1,5 V à +1,5 V, une vitesse de balayage dans la plage de 0,02 V/s à 0,5 V/s, et un nombre de cycles dans la plage de 5 à 100.

6. - Procédé selon la revendication 5, comprenant la sélection d'une durée d'électropolymérisation dans la plage de 30 minutes à 2 heures.

7. - Procédé selon l'une quelconque des revendications 5 ou 6, dans lequel la vitesse de balayage est de 0,1 V/s.

8. - Procédé selon l'une quelconque des revendications 1 à 7, comprenant l'utilisation d'un monomère contenant de la dopamine en tant que monomère pour le revêtement par ladite couche de polymère.

9. - Procédé selon la revendication 9, comprenant l'utilisation de dopamine HCl en tant que monomère.

10. - Procédé selon l'une quelconque des revendications 5 à 9, comprenant l'ajustement de la concentration en monomère dans l'électrolyte pour être de 1 mg/mL au début de l'électropolymérisation, et la préparation de l'électrolyte pour qu'il soit une solution conductrice tamponnée.

11. - Procédé selon la revendication 10, dans lequel l'électrolyte est tamponné à un pH de 7,4.

12. - Procédé selon l'une quelconque des revendications 5 à 11, dans lequel Ag-AgCl est utilisé en tant que matériau d'électrode et Pt est utilisé en tant que matériau de contre-électrode.
